# EUROPEAN PATENT SPECIFICATION

(11) **EP 1 637 101 B1**
(45) Date of publication and mention of the grant of the patent: **21.11.2007**
(21) Application number: 05111600.2
(22) Date of filing: 23.12.2002
(51) Int. Cl.: A61F 9/01, A61F 9/013, A61F 2/16

(54) **Adaptive wavefront method for ophthalmic surgery**
Adaptives Wellenfront-Modulationssystem und Verfahren zur refraktiven Augenoperation
Système de modulation adaptive par front d'ondes et méthode pour la chirurgie ophtalmique

(30) Priority: 14.01.2002 US 348812 P; 20.05.2002 US 151404; 06.09.2002 US 236375
(43) Date of publication of application: 22.03.2006
(62) Divisional of application: 02028928.6
(73) Proprietor: Alcon, Inc., 6331 Hünenberg (CH)
(72) Inventor: Sheets, John W., Bridgewater, NJ 08807-2391 (US); Campin, John A., FL 32828, Orlando (US); Pettit, George, FL 32751, Maitland (US)
(74) Representative: Hanna, Peter William Derek

(56) References cited:
- WO-A-01/28410
- US-A- 4 669 466
- US-A- 6 086 204
- US-A- 6 149 643

## Description

### Background of the Invention

The present invention relates to methods associated with performing corrective surgery on the eye, and, more particularly, to such methods that adaptively modulate sensed data on the basis of data from prior procedures.

In conventional refractive laser surgery a clinician typically modifies a prescription entered into the treatment system. Such modifications are based upon prior experience with outcomes achieved with that particular treatment system, and also upon experience with particular patient populations derived from, for example, demographic data. For example, a surgeon might enter a 2-diopter myopic treatment prescription for a patient diagnosed with 3 diopters of myopia if analysis of previous outcomes indicates a 50% overcorrection using this system for patients of a particular category. Such an empirical alteration of entered treatment parameters based upon previous experience is referred to as a nomogram adjustment. Nomograms are considered essential by the ophthalmic community because different clinicians employ different surgical techniques, operate under different environmental conditions, have distinct patient demographics, etc.

Conventional surgery involves a limited number of well-defined treatment parameters, principally spherical error, astigmatic error, astigmatic axis, optical zone size, and blend zone size. Thus it is relatively straightforward for a surgeon to develop nomogram formulas based on conventional clinical examinations before and after surgical procedures. In contrast, wavefront-guided customized treatments, such as that disclosed in commonly owned U.S. Patent No. 6,270,221 B1, involve complex a mathematical description of the pre-operative aberration profile, which is transferred electronically to the treatment system.

Although such a precise wavefront description can in theory be modified empirically to yield a better outcome, typically clinicians are not skilled in the analytic interpretations of these mathematical parameters. In addition, at present there is no known convenient method for a surgeon to modify a wavefront-based prescription prior to a procedure such as laser surgery.

In currently used wavefront-based treatments, the raw wavefront data are modulated to generate a treatment profile in order to account for an apparent radial dependence in the effectiveness of ablative treatment on the corneal tissue. This, however, is currently applied identically in all treatments.

Prior art document US 6,086,204 (D1) discloses methods and devices to design and fabricate surfaces on contact lenses that correct the eye's optical aberrations. Prior art documents WO 01 28410 and US 6 149 643 refer to cornea reshaping methods and systems and although they consider the past experience, they do not provide a clear and quantitative way of taking this experience into account.

### Brief Summary of the Invention

The present invention provides a method in accordance with the following claim, in particular suitable for creating a nomogram for adaptively modulating sensed wavefront data based upon prior treatment outcomes.

It is a further object to provide such a method that is site-specific.

It is another object to provide such a method that is demographically based.

These and other objects are achieved by the present invention, one aspect of which is a method for refining a prescription for an intraocular lens or for phakic refractive lens implant. The method comprises the steps of receiving a measured correction prescription for a current patient. Typically the prescription will have been obtained using a wavefront determination. The current patient will have associated with him/her a classification element for placing the patient in at least one particular category.

Next a database of treatment outcomes on a plurality of previously treated patients is accessed. The database contains, for each previously treated patient, at least one classification element and also comprises a preoperative wavefront-determined correction prescription and a postoperative visual profile. A difference between the preoperative correction prescription and the postoperative visual profile represents an over- or undercorrection resulting from the surgery.

Treatment outcome data are accessed from the database based upon possessing a classification element in common with the current patient. From these data, an average difference may be calculated between the preoperative prescription and the postoperative profile. This average difference is then used to adjust the current patient's correction prescription to form an optimized prescription prior to performing the procedure or the manufacture or customization of any lens implant.

A further aspect includes a method for creating a system for optimizing a prescription for a lens implant, which includes the steps of forming a database of treatment outcomes as described above. A search engine resident on a processor is adapted to extract treatment outcomes based upon a classification element. Software is also provided for performing the calculational steps as outlined above.

The features that characterize the invention, both as to organization and method of operation, together with further objects and advantages thereof, will be better understood from the following description used in conjunction with the accompanying drawing. The drawings are for the purpose of illustration and description. These and other objects attained, and advantages offered, by the present invention will become more fully apparent as the description that now follows is read in conjunction with the accompanying drawing.

### Brief Description of the Drawings

FIG. 1 is a schematic diagram of the system of the present invention.
FIGS. 2A and 2B is a flow chart of a method for optimizing a treatment prescription for a current patient.
FIG. 3 illustrates an exemplary algorithm for calculating optimized coefficients for a treatment prescription.
FIG. 4 is a flow chart of a method for creating a database of treatment outcomes.

### Detailed Discription of the Invention

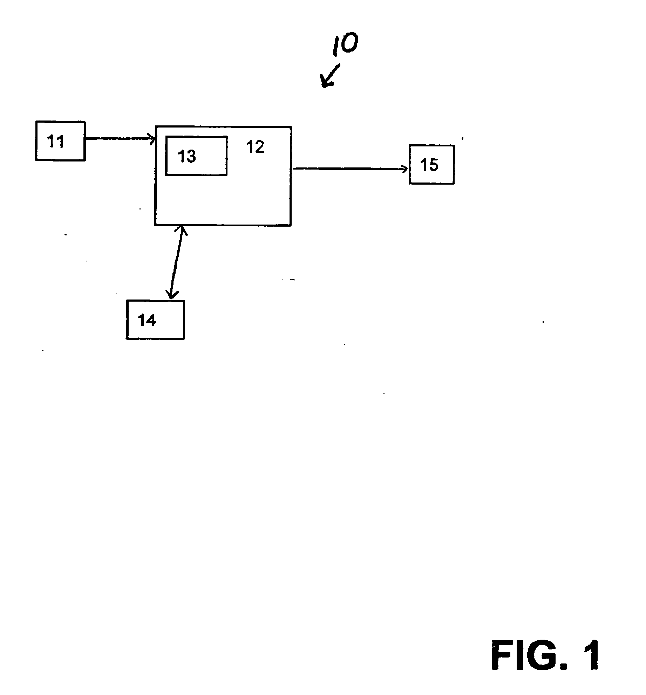

A description of the preferred embodiments of the present invention will now be presented with reference to FIGS. 1-4.

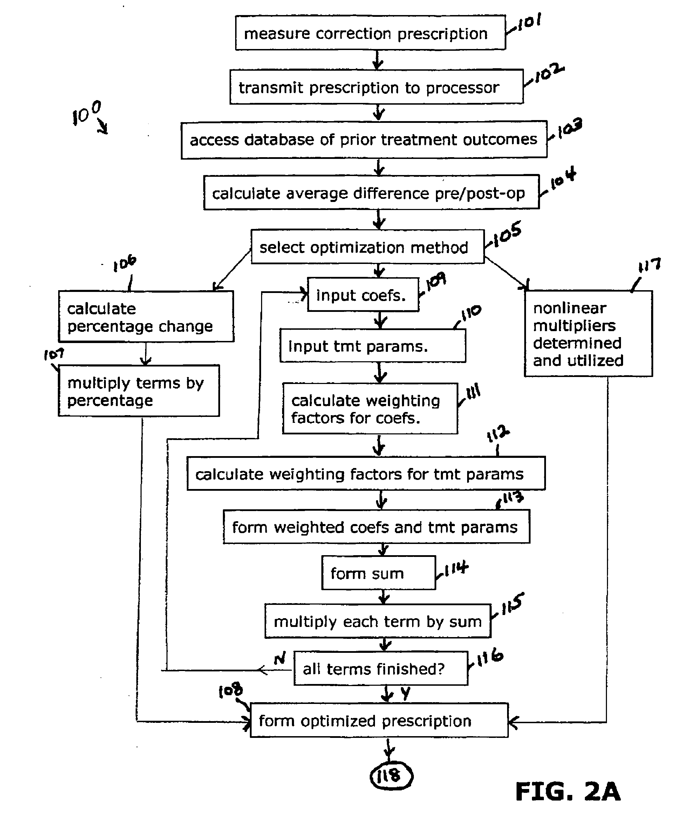

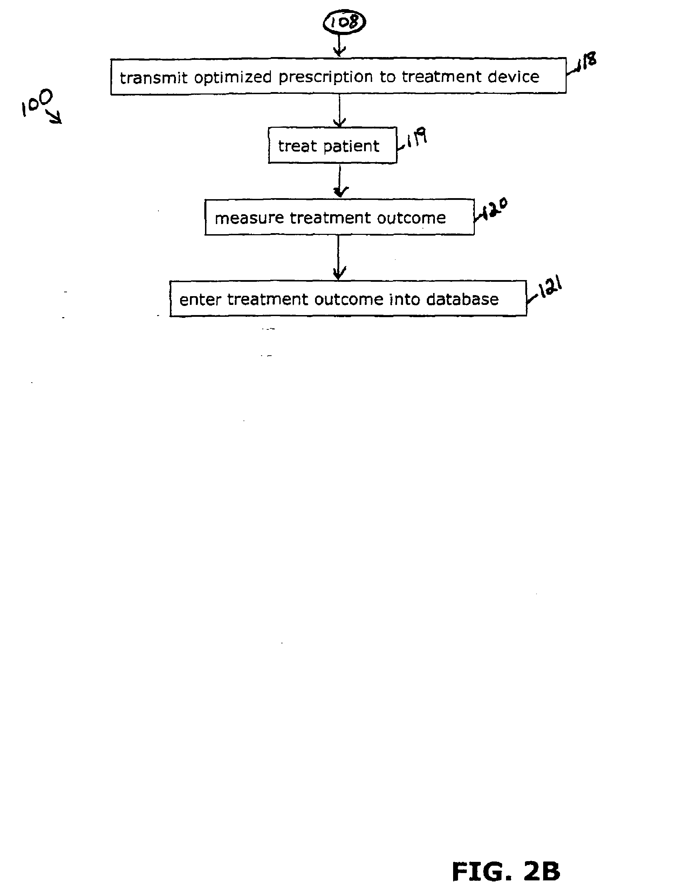

Method 100 (FIGS. 2A, 2B) of the present invention is directed to an optimization of a prescription for ophthalmic lens or implant. In a preferred embodiment a measured correction prescription is measured (block 101) using wavefront determination apparatus 11 for a current patient. Typically the correction prescription comprises an algorithm having a plurality of terms, each of which has associated therewith a coefficient. For example, the wavefront may be described mathematically using a standardized form, such as Zernike polynomials, Taylor polynomials, or Fourier series, although these are not intended as limitations. For any such form describing a mathematical shape, a specific wavefront can be described by the numerical values for the weighting of the various terms in the mathematical expression.

The raw correction prescription is received into processor 12 housing software package 13 for a current patient (block 102) having at least one uniquely associated classification element. Among the classification elements may be included such data as, but not intended to be limited to, patient-specific data, such as age, gender, and ethnic background, and site-specific data such as local elevation and environmental parameters such as temperature or humidity.

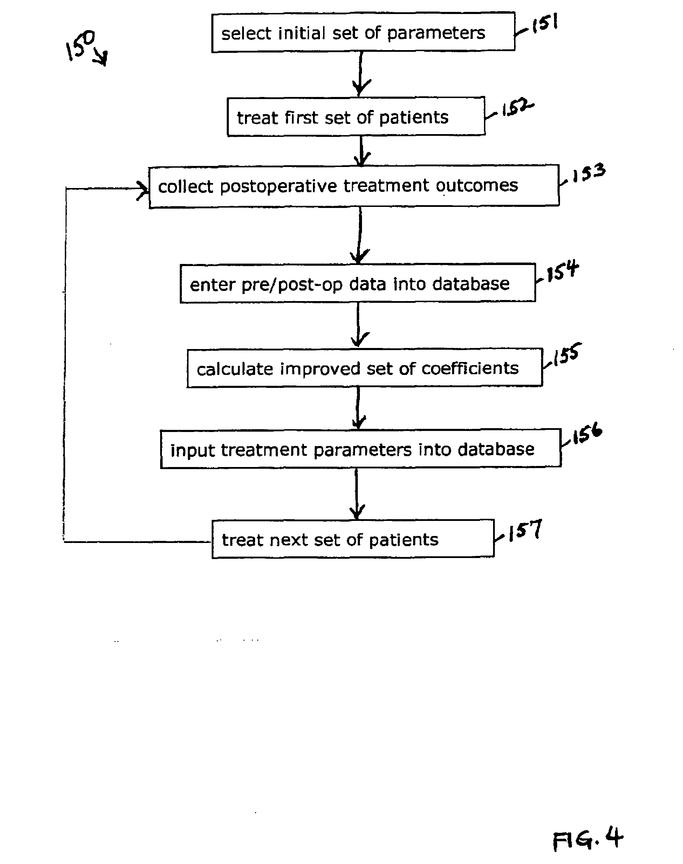

Database 14 of treatment outcomes on a plurality of previously treated patients, which is created in steps such as illustrated in FIG. 4, is accessed (block 103) by software package 13. Each treated patient outcome has associated therewith at least one classification element and comprises a preoperative wavefront-determined correction prescription and a postoperative visual profile.

From the treatment outcomes in database 14 is calculated an average difference between the preoperative prescription and the postoperative profile for at least some of the previously treated patients having a classification element in common with the current patient (block 104). As preferred embodiments only, three methods for achieving an optimized prescription from this calculation step will be presented herein (block 105). In first method 100, illustrated in FIGS. 2A and 2B, a linear scaling adjustment, the calculating step further comprises calculating from the average difference a percentage difference (block 106). The current patient correction prescription is then adjusted commensurate with the calculated average difference to form an optimized prescription, thereby avoiding a statistically calculable over or undercorrection. In method 100, the adjusting step comprises multiplying the algorithm terms by the percentage difference (block 107), globally increasing or decreasing the wavefront profile, to form the optimized prescription (block 108).

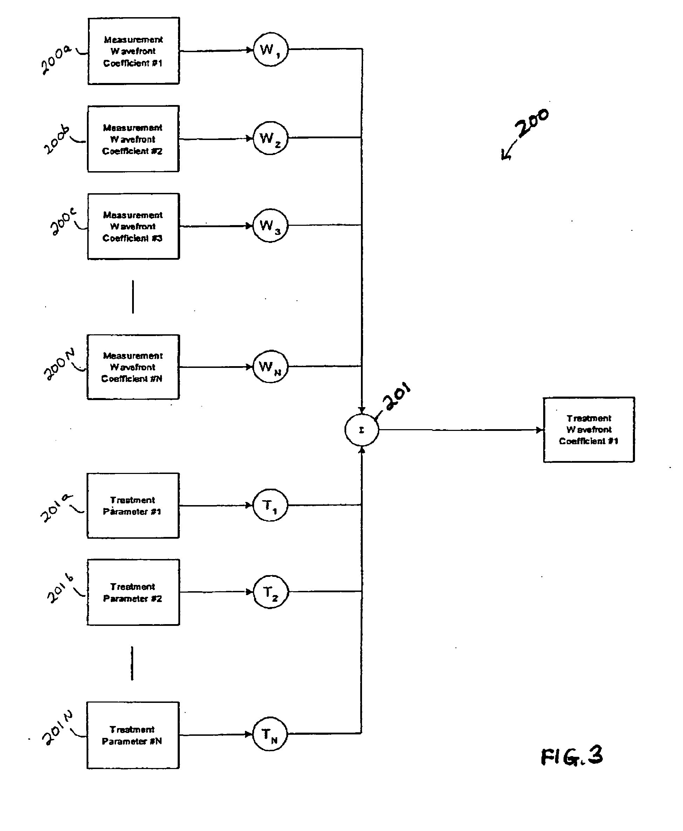

In second method 300, an algorithm for which is illustrated in FIG. 3, and the flow chart for which is shown in FIGS. 2A and 2B, a "nomogram"-type approach is used wherein an object of the optimization procedure is to arrive at a modified description of the measured wavefront, using the same mathematical notation scheme as used in determining the correction prescription. The goal of the modified description is to achieve an optimal treatment outcome when used to calculate the actual ablation treatment profile to be used on the patient.

In FIG. 3 is illustrated how algorithm 200 of method 300 arrives at an optimized value for one output coefficient. In this aspect of method 300, the data set input to algorithm 200 includes the true coefficients of the measured wavefronts 200a, 200b, 200c, ..., 200N (block 109, FIG. 2A). Additional input data include input values for other treatment parameters 201 a, 201 b, ..., 201 N (block 110). The treatment parameters may comprise such data as patient demographic parameter, such as age, gender, or ethnicity; a site-specific environmental parameter, such as site altitude, temperature, or humidity; and a flap parameter, such as expected flap thickness or hinge location.

In algorithm 200, the calculating step then further comprises converting the calculated average difference into a weighting factor, shown in FIG. 3 as W1, W2, W3, ..., WN for each of the coefficients associated with the wavefront determination algorithm terms (block 111), and also converting the calculated average difference into a weighting factor for the one treatment parameters, shown in FIG. 3 as T1, T2, ..., TN (block 112). The adjusting step comprises multiplying each coefficient and treatment parameter by the respective weighting factor to form weighted coefficients and weighted treatment parameters (block 113). Next the weighted coefficients for each term and the weighted treatment parameters are summed (block 114; " SIGMA " 201 in FIG. 3), and each term is multiplied by the sum of the weighted coefficients and weighted treatment parameters (block 115).

This procedure (blocks 109-115) is continued for all terms in the wavefront description (block 116) until a complete optimized prescription is formed (block 108).

It will be understood by one of skill in the art that this particular embodiment represents an exemplary method, and that alternate embodiments may be envisioned without departing from the spirit of the invention. For example, in a third embodiment (FIG. 2A), a nonlinear approach may be utilized wherein at least some the weighting coefficients are not simple linear multipliers (block 117), such as coefficients that change depending upon the input value, or are influenced by other factors in an interdependent manner. As system 10 and methods 100 and 300 are conceived to be adaptive, it will be appreciated by one of skill in the art that an algorithm that "learns" from new input data is possible when the database has sufficient data therein from which to form statistically valid correlations.

Once an optimized prescription is determined (block 108) from any of the methods, the optimized prescription may be automatically transmitted to treatment device 15 (block 118, FIG. 2B) or implant manufacturing device 16. Alternatively, the calculations may be made within the processor 12 following transmission of the raw prescription data to the treatment device 15 or implant manufacturing device 16.

Preferably, following each treatment (block 119) of a current patient, a treatment outcome on the current patient is measured (block 120) at a predetermined interval following the treatment. In order to continuously enrich the database, the treatment outcome for the current patient is then entered into the database (block 121)

Figure 4 discloses a method 150 for the creation of a system from which to extract optimization data for use in previously described method 100. As shown in FIG. 4, an initial set of parameters are selected (block 151), with the weighting coefficients set to nominal values. For example, the weights may be set to translate the measurement wavefront directly into the treatment wavefront without modification. In FIGS. 2A and 2B, this would correspond to W1 220a being equal to 1 and all other terms being equal to 0 for determining the first treatment wavefront coefficient.

Using this initial set of parameters, a first set of patients are treated (block 152), and postoperative treatment outcomes are collected after a predetermined interval (block 153). The pre- and postoperative data, along with the associated classification element(s), are entered into a database 14 (block 154).

Search engine 18 resident on the processor 12 is adapted to extract treatment outcomes based upon a classification element desired for correlation calculations. As above, an improved set of coefficients can then be calculated (block 155) for a second set of patients using these data.

Treatment outcomes from the second set of patients are then entered into database 14 (block 156), thereby further improving the statistics for the data. This process can be continued with a next set of patients (block 157), and further continued essentially indefinitely, shown by the return arrow to block 153 in FIG. 4, to further refine the adjustment algorithm.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the system and method illustrated and described herein are by way of example.

## Claims

1. A method (100) for creating a system for optimizing a prescription for refractive correction, comprising the steps of:
a) forming a database (14) of treatment outcomes on a plurality of treated patients, each treated patient outcome having associated therewith at least one classification element and comprising a preoperative wavefront-determined (11) correction prescription and a postoperative visual profile;
b) providing a search engine (16) communicating with a processor (12) adapted to extract treatment outcomes based upon a classification element;
c) providing a first software module (13) for use by the processor, the first software module adapted to calculate (104) from the extracted treatment outcomes in the database an average difference between the preoperative prescription and the postoperative profile for at least some of the previously treated patients having a classification element in common with the current patient;
d) providing a second software module (13) for use by the processor, the second software module adapted to adjust a current patient correction prescription commensurate with the calculated average difference to form (108) an optimized prescription, the current patient having a classification element associated therewith and having had a wavefront determination made for measuring an initial correction prescription, and
e) providing means for transmitting (118) the optimized prescription automatically to an implant manufacturing device (15).

## Patentansprüche

1. Verfahren (100) zum Erzeugen eines Systems zur Optimierung einer Verordnung einer Refraktionskorrektur, das folgende Schritte aufweist:
a) Bilden einer Datenbank (14) aus Behandlungsergebnissen von mehreren behandelten Patienten, wobei jedes Ergebnis eines behandelten Patienten wenigstens ein diesem zugeordnetes Klassifizierungselement besitzt und eine Korrekturverordnung durch präoperative Wellefrontbestimmung (11) und ein postoperatives visuelles Profil aufweist,
b) Bereitstellen einer Suchmaschine (16), die mit einem Prozessor (12) in Verbindung steht, der dafür eingerichtet ist, Behandlungsergebnisse aufgrund eines Klassifizierungselements auszuwählen;
c) Bereitstellen eines ersten Softwaremoduls (13) zur Verwendung durch den Prozessor, wobei das erste Softwaremodul dafür eingerichtet ist, aus den ausgewählten Behandlungsergebnissen in der Datenbank eine mittlere Differenz zwischen der präoperativen Verordnung und dem postoperativen Profil für wenigstens einige der vorhergehend behandelten Patienten zu berechnen (104), die ein Klassifizierungselement mit dem aktuellen Patienten gemein haben;
d) Bereitstellen eines zweiten Softwaremoduls (13) zur Verwendung durch den Prozessor, wobei das zweite Softwaremodul dafür eingerichtet ist, eine Korrekturverordnung für den aktuellen Patienten entsprechend der berechneten mittleren Differenz abzustimmen, um eine optimierte Verordnung zu bilden (108), wobei der aktuelle Patient ein Klassifizierungselement besitzt, das diesem zugeordnet ist, und eine Wellefrontbestimmung hatte, die für eine Messung einer anfänglichen Korrekturverordnung vorgenommen wurde; und
e) Bereitstellen von Mitteln zum automatischen Übertragen (118) der optimierten Verordnung an eine Vorrichtung zur Implantatherstellung (15).

## Revendications

1. Procédé (100) pour créer un système pour optimiser une prescription pour une correction de réfraction, comportant les étapes consistant à :
a) former une base de données (14) de résultats de traitement concernant une pluralité de patients traités, chaque résultat de patient traité étant associé à au moins un élément de classification et comportant une prescription de correction (11) déterminée par front d'ondes pré-opératoire et un profil visuel post-opératoire,
b) fournir un moteur de recherche (16) communiquant avec un processeur (12) adapté pour extraire des résultats de traitement sur la base d'un élément de classification,
c) fournir un premier module logiciel (13) pour une utilisation par le processeur, le premier module logiciel étant adapté pour calculer (104) à partir des résultats de traitement extraits de la base de données une différence moyenne entre la prescription pré-opératoire et le profil post-opératoire pour au moins une partie des patients précédemment traités ayant un élément de classification en commun avec le patient courant,
d) fournir un second module logiciel (13) pour une utilisation par le processeur, le second module logiciel étant adapté pour ajuster une prescription de correction de patient courante comparable à la différence moyenne calculée pour former (108) une prescription optimisée, le patient courant ayant un élément de classification qui lui est associé et ayant une détermination de front d'onde réalisée pour mesurer une prescription de correction initiale, et
e) fournir des moyens pour transmettre (118) la prescription optimisée automatiquement à un dispositif de fabrication d'implant (15).
